# EUROPEAN PATENT APPLICATION

(11) **EP 4 524 983 A2**
(43) Date of publication of application: **19.03.2025**
(21) Application number: 24200653.4
(22) Date of filing: 16.09.2024
(51) Int. Cl.: G16H 20/17, G16H 40/20, G16H 40/60, G16H 40/63

(54) **LOCATION BASED ADAPTIVE MEDICAL DEVICE BEHAVIOR**

(30) Priority: 18.09.2023 US 202363538912 P
(71) Applicant: Hill-Rom Services, Inc., Batesville, IN 47006-9167 (US)
(72) Inventor: MONSON, Gavin, Batesville, 47006-9167 (US); NEWKIRK, David C., Batesville, 47006-9167 (US)
(74) Representative: Reddie & Grose LLP

(57) **Abstract**

An adaptive healthcare system (10) includes at least one medical device (12A-12N) having a plurality of settings. A monitoring system (14) includes a device location identifier (16A-16N) and a plurality of caregiver location identifiers (18A-18N) that include at least one of a first classification (C1) and a second classification (C2). The adaptive healthcare system (10) further includes processor (104) and memory (106) containing instructions that when carried out by the processor (104), cause the processor (104) to perform various functions. More particularly, the processor (104) is caused to detect that a proximity between the device location identifier (16A-16N) and one of the plurality of caregiver location identifiers (18A-18N) is within a predetermined range (D1-DN) and determine if the detected caregiver location identifier (18A-18N) includes the first classification (C1) or the second classification (C2). The processor (104) is further caused to enable at least one of the plurality of settings of the medical device (12A-12N) based on a combination of the proximity and the determined classification (C1, C2) of the detected caregiver location identifier (18A-18N).

## Description

### FIELD OF THE DISCLOSURE

The present disclosure generally relates to an adaptive healthcare system, and, more particularly, to an adaptive healthcare system that includes a device location identifier and a plurality of caregiver location identifiers that permit or restrict settings of a medical device based on a combination of the proximity and the determined classification.

### BACKGROUND

In a medical environment, several caregivers may be tasked with the care of a patient, generally, certain caregivers have specific duties and responsibilities related to the care of the patient. The specific duties and responsibilities may be limited based on the caregiver's education, licensing, and other factors. These limitations may be based, at least in part, on which caregivers have responsibilities to utilize certain medical devices. More particularly, specialized caregivers may have unrestricted usage of, for example, drug delivery devices or other specialized medical devices. On the other hand, less experienced caregivers may not be permitted to utilize certain medical devices or settings of the medical devices. As such, monitoring the medical environment and ensuring that usage of the medical devices is limited to only those caregivers with specialized training, can be difficult.

Accordingly, the present disclosure generally relates to an adaptive healthcare system that includes a device location identifier and a plurality of caregiver location identifiers that permit or restrict the settings of a medical device based on a combination of the proximity and the determined classification.

### SUMMARY OF THE DISCLOSURE

According to one aspect of the present disclosure, an adaptive healthcare system includes at least one medical device having a user interface with a plurality of settings, the user interface includes a power saving mode and a power unrestricted mode. A monitoring system includes a device location identifier and a plurality of caregiver location identifiers that include at least one of a first caregiver classification or and second caregiver classification. The adaptive healthcare system further includes a processor and memory containing instructions that, when carried out by the processor, cause the processor to perform various functions. More particularly, the processor is caused to detect that a proximity between the device location identifier and one of the plurality of caregiver location identifiers is within a predetermined range and switches the user interface from the power saving mode to the power unrestricted mode. The processor is further caused to determine if the detected caregiver location identifier includes the first caregiver classification or the second caregiver classification. The processor is further caused to enable at least one of the plurality of settings of the medical device based on a combination of the proximity and the determined classification of the detected caregiver location identifier.

According to another aspect of the present disclosure, an adaptive healthcare system for a medical facility having a room includes a visual indicator configured to be located in the room and to communicate a need of a patient. A medical device is configured to be located in the room and includes a plurality of settings. A monitoring system includes a device location identifier and a plurality of personnel location identifiers that each include one of a plurality of classifications. The adaptive healthcare system further includes a processor and memory, the memory containing instructions that when carried out by the processor, cause the processor to detect that a proximity between the device location identifier and one of the plurality of personnel location identifiers is within a predetermined range that is within view of the visual indicator. The processor is further caused to determine that the personnel location identifier includes a classification with a responsibility associated with the need of the patient and illuminate the visual indicator while the detected personnel location identifier is within the predetermined range.

According to yet another aspect of the present disclosure, an adaptive healthcare system includes a medical device including a plurality of settings. A monitoring system includes a device location identifier and a pair of personnel location identifiers that include a caregiver classification and an oversight classification. The adaptive healthcare system further includes a processor and memory, the memory containing instructions that, when carried out by the processor, cause the processor to detect that a proximity between the device location identifier and the personnel location identifier associated with the caregiver classification is within a predetermined range. The processor is further caused to enable a restricted plurality of settings of the medical device based on a combination of the proximity and the caregiver classification and detect if a proximity between the device location identifier and the personnel location identifier associated with the oversight classification is within a predetermined range. If the personnel location identifier associated with the oversight classification is within a predetermined range, the processor enables additional plurality of settings of the medical device.

These and other features, advantages, and objects of the present disclosure will be further understood and appreciated by those skilled in the art by reference to the following specification, claims, and appended drawings.

### BRIEF DESCRIPTION OF THE DRAWINGS

In the drawings:
FIG. 1 is a front perspective view of an adaptive healthcare system with a patient table, according to an aspect of the present disclosure;
FIG. 2 is a side elevational view of an adaptive healthcare system within a medical facility, according to an aspect of the present disclosure;
FIG. 3 is a top, plan view of an adaptive healthcare system within a medical facility, according to an aspect of the present disclosure;
FIG. 4 is a schematic view of a plurality of predetermined ranges between an individual and a medical device that facilitates providing or restricting certain settings associated with a medical device, according to an aspect of the present disclosure;
FIG. 5A is a schematic view of a control system of an adaptive healthcare system, according to an aspect of the present disclosure;
FIG. 5B is a top plan view of a computer program that creates a visual reconstruction of a medical environment surrounding a medical incident, according to an aspect of the present disclosure; and
FIG. 6 is a flow chart of a method of utilizing an adaptive healthcare system, according to an aspect of the present disclosure.

### DETAILED DESCRIPTION

The present illustrated examples reside primarily in combinations of method steps and apparatus components related to an adaptive healthcare system that includes a device location identifier and a plurality of caregiver location identifiers that permit or restrict settings of a medical device based on a combination of the proximity and the determined classification. Accordingly, the apparatus components and method steps have been represented, where appropriate, by conventional symbols in the drawings, showing only those specific details that are pertinent to understanding the examples of the present disclosure so as not to obscure the disclosure with details that will be readily apparent to those of ordinary skill in the art having the benefit of the description herein. Further, like numerals in the description and drawings represent like elements.

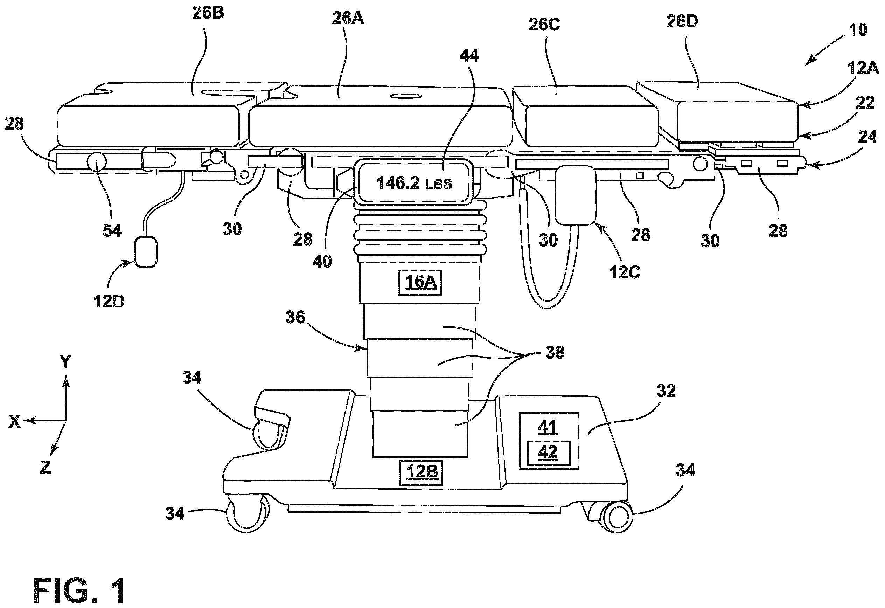

For purposes of description herein, the terms "upper," "lower," "right," "left," "rear," "front," "vertical," "horizontal," and derivatives thereof, shall relate to the disclosure as oriented in FIG. 1. Unless stated otherwise, the term "front" shall refer to a surface closest to an intended viewer, and the term "rear" shall refer to a surface furthest from the intended viewer. However, it is to be understood that the disclosure may assume various alternative orientations, except where expressly specified to the contrary. It is also to be understood that the specific structures and processes illustrated in the attached drawings, and described in the following specification are examples of the inventive concepts defined in the appended claims. Hence, specific dimensions and other physical characteristics relating to the examples disclosed herein are not to be considered as limiting, unless the claims expressly state otherwise.

The terms "including," "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus. An element preceded by "comprises a ... " does not, without more constraints, preclude the existence of additional identical elements in the process, method, article, or apparatus that comprises the element.

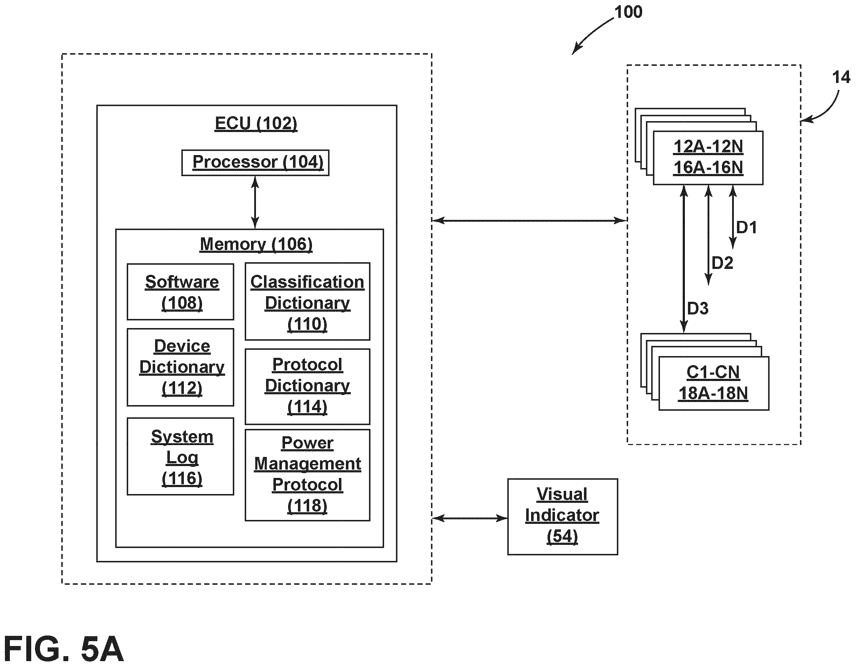

Referring to FIGS. 1-6, reference numeral 10 generally designates an adaptive healthcare system. The adaptive healthcare system 10 includes at least one medical device 12A-12N having a plurality of settings. A monitoring system 14 includes a device location identifier 16A-16N (e.g., a first medical device identifier 16A) and a plurality of personnel location identifiers 18A-18N (e.g., caregiver location identifiers 18A-18N) that includes at least one of a first caregiver classification C1 and a second caregiver classification C2. The adaptive healthcare system 10 further includes a control system 100 (FIG. 5A) that includes a processor 104 and memory 106 containing instructions that, when carried out by the processor 104, cause the processor 104 to perform various functions. More particularly, the processor 104 is caused to detect that a proximity between the device location identifier 16A-16N and one of the plurality of caregiver location identifiers 18A-18N is within a predetermined range D1-DN and determine if the detected caregiver location identifier 18A-18N includes the first caregiver classification C1 or the second caregiver classification C2. The processor 104 is further caused to enable at least one of the plurality of settings of the medical device 12A-12N based on a combination of the proximity and the determined classification C1, C2 of the detected caregiver location identifier 18A-18N.

With reference now to FIG. 1, the at least one medical device 12A-12N may include a patient table 12A with a variety of settings. The patient table 12A may include a table, bed, or stretcher, for example, to move the patient, for the patient to rest, or for providing care (e.g., surgery, drug deliver, and/or the like). The patient table 12A may include a patient support mattress 22 and a mattress support frame 24 that supports and positions the patient support mattress 22. In some examples, the patient support mattress 22 includes one or more (e.g., two, three, four, five, or six) pads 26A-26D (e.g., in the same or a separate envelope or outer casing) and the mattress support frame 24 may include one or more sub-support frames 28 (e.g., two, three, four, five, or six). The sub-support frames 28 may be operably coupled by linkages 30 that permit articulation between adjacent sub-support frames 28 to position a patient in varying orientations. In some examples, the one or more pads 26A-26D may be integrally (e.g., permanently or with additional tools and/or fasteners) connected with the sub-support frames 28 such that they cannot be removed after assembly. In other examples, the one or more pads 26A-26D may be non-integrally (e.g., removable via Velcro, ties, and/or the like) connected with the sub-support frames 28 such that they can be connected to and removed from the sub-support frames 28. It should also be appreciated that select pads 26A-26D may be integrally connected and select pads 26A-26D may be non-integrally connected within the patient table 12A. The non-integral connection may be beneficial to remove the one or more pads 26A-26D for cleaning, replacement, or servicing. The pads 26A-26D may include a central pad 26A, located centrally to support a midsection of a patient, a top pad 26B to provide support for a head of a patient, and lower pads 26C and 26D to support the legs of a patient (e.g., on either side of the knee). The patient table 12A further includes a base frame 32 that may include a plurality of caster wheels 34 that allow the patient table 12A to roll between different locations (e.g., along an X-axis and a Z-axis). A central pedestal 36 operably connects the base frame 32 to the mattress support frame 24. The central pedestal 36 may be vertically (e.g., along a Y-axis) adjustable between a variety of positions to suit the needs of a caregiver. More particularly, the central pedestal 36 may include a series of telescopically adjustable sleeves. In some examples, the central pedestal 36 connects centrally on the base frame 32 between the caster wheels 34 and centrally on the mattress support frame 24, such that a patient's center of mass is substantially aligned therewith along the Y-axis. In some examples, a central wheel (not shown) may be located under the central pedestal 36 to facilitate movement. The central wheel may be driven via a motor, and/or the like, and may be moveable between an operating (i.e., rolling) position and a stowed position.

With continued reference to FIG. 1, the at least one medical device 12A-12N (e.g., the patient table 12A) may include a user interface 40 for selecting the various settings, such as the central pedestal 36 positioning, locking, unlocking, and moving the caster wheels 34, and/or the like. The patient table 12A may have a power system 41 such as a wall outlet plug, a battery 42, or a hybrid combination of both that powers the user interface 40 and operation of the various settings. In addition, it should be appreciated that the patient table 12A may include additional settings and features. For example, the patient table 12A may include, be operably connected, or separate from various other medical devices 12B-12N. In some examples, some or all of the other medical devices 12B-12N may be operable through the user interface 40 and powered by the battery 42. In some examples, some or all of the other medical devices 12B-12N may be operable through independent and separate user interfaces and power supplies (e.g., wired and/or battery). The user interface 40 may include a power saving mode (e.g., wherein the user interface 40 requires less power) and a power unrestricted mode (e.g., wherein the user interface 40 requires more power). The user interface 40 may typically operate in the power saving mode until a caregiver location identifier C1, C2 is within a predetermined range. In this manner, the control system 100 may further be configured to, upon detecting one of the caregiver location identifiers C1, C2 is within the predetermined range, switching the user interface 40 from the power saving mode to the power unrestricted mode.

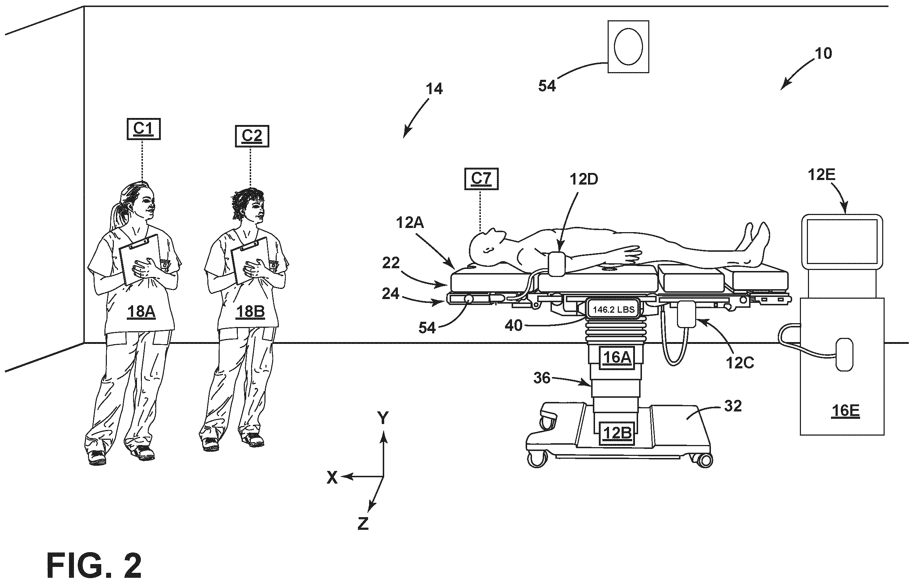

With reference now to both FIGS. 1 and 2, the other medical devices 12B-12N may include a weight sensor 12B. The weight sensor 12B may be located in and/or generally aligned with the central pedestal 36 along the Y-axis. The weight sensor 12B may selectively display the weight of the patient on display 44 of the user interface 40. The weight sensor 12B may be powered by the battery 42. In some examples, the other medical devices 12B-12N may include a vitals monitor 12C. The vitals monitor 12C may be directly connected to and/or integrated with components of the patient table 12A. The vitals monitor 12C may selectively monitor various vitals of the patient. For example, the vitals may include a heart rate, oxygen level, blood pressure, respiration rate, and temperature. The measurement of the vitals may be selectively generated on the display 44 of the user interface 40. The measurement and display of the vitals may be controlled by operating the various settings on the user interface 40. The vitals monitor 12C may be powered by the battery 42. In some examples, the other medical devices 12B-12N may include a fluid delivery system 12D. The fluid delivery system 12D may be directly connected to and/or integrated with components of the patient table 12A. The fluid delivery system 12D may selectively provide fluids (e.g., water, electrolytes, etc.) to the patient. A measurement of the fluid exchange may be generated on the display 44 of the user interface 40 and/or a different user interface. The rate and display of the fluid exchange may be controlled by operating the various settings on the user interface 40. The fluid delivery system 12D may be powered by the battery 42 and/or an independent power source. In some examples, the other medical devices 12B-12N may include a drug delivery system 12E. The drug delivery system 12E may be directly connected to and/or integrated with components of the patient table 12A. The drug delivery system 12E may selectively provide medicines, anesthetics, and/or the like to the patient. A measurement of the medicine delivery provided to the patient may be generated (e.g., monitored, transmitted, and visualized) on the display 44 of the user interface 40 and/or on a different display. The rate of delivery and display of the fluid exchange may be controlled by operating the various settings on the user interface 40 and/or a different user interface. The drug delivery system 12E may be powered by the battery 42 and/or an independent power source. It should be appreciated that the example medical devices 12A-12E are exemplary in nature and that other medical devices 12N may be used within the adaptive healthcare system 10. For example, ventilators, blood transfusion, and other medical devices (e.g., with delivery systems and delivery rates) critical to the support of the patient, like the drug delivery system 12E, may utilize the adaptive healthcare system 10 and functions associated therewith. In some examples, each medical device 12A-12N may be associated with a unique device location identifier 16A-16N, such that the adaptive healthcare system 10 can monitor the use of each medical device 12A-12N individually. In some examples, each medical device 12B-12N includes a power system like the power system 41 in the patient table 12A.

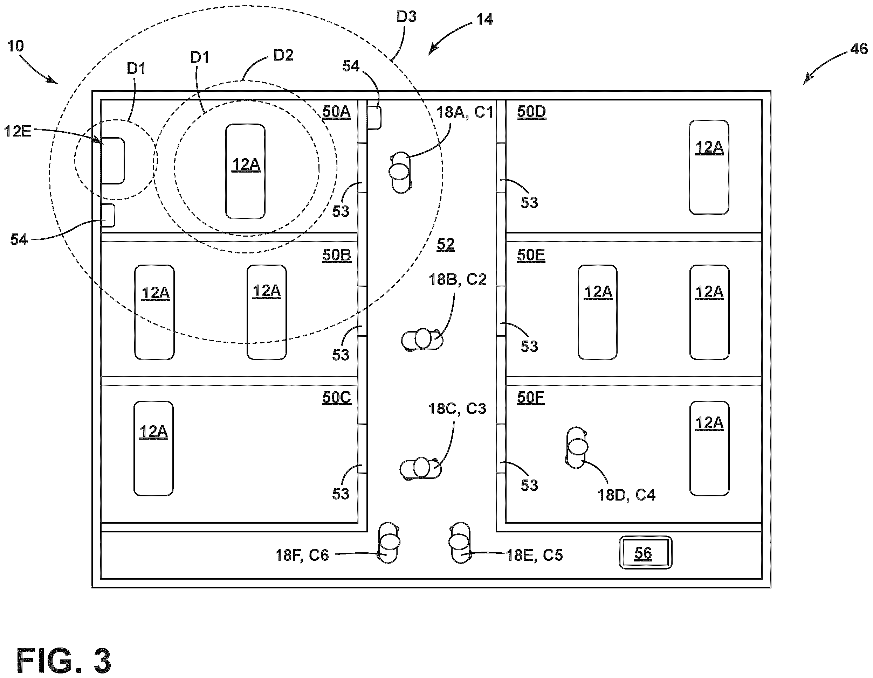

With reference now specifically to FIGS. 2 and 3, the medical devices 12A-12N may be located in a medical facility 46, such as a hospital, that employs several caregivers with different responsibilities for monitoring and providing care to the patient specific ones of the medical devices 12A-12N. In addition, the medical facility 46 may include several visitors and other staff members who do not have direct patient care responsibilities. Generally speaking, the adaptive healthcare system 10 facilitates providing classifications C1-CN, including the first caregiver classification C1 and the second caregiver classification C2 that provide access to various settings of the medical devices 12A-12N based on the associated caregiver assigned responsibilities. For example, some caregivers may be licensed and/or otherwise permitted to utilize certain features of the medical devices 12A-12N that another, untrained or unlicensed, caregiver is not permitted to use. More particularly, various types of treatments, such as drug delivery, are typically assigned to specialty caregivers rather than each caregiver in the medical facility 46. In addition to the first and second caregiver classifications C1, C2, there may be additional classifications C3-CN, such as a third caregiver classification C3, a visitor classification C4, a non-care providing staff classification C5, a priority user classification C6, and a variety of additional classifications CN related to responsibility and permission to use the settings of the various medical devices 12A-12N. Each classification C1-CN may correspond with a different location identifier 18A-18N. In this manner, the certain classifications C1, C2, and C5 may have access to certain settings of one or more of the medical devices 12A-12N based at least in part to the proximity of the location identifier 18A-18N to the medical device 12A-12N and the classification C1-CN. In other words, location identifiers 18A-18N are associated with classification C1-CN, which, in turn, is associated with permissions to the medical devices 12A-12N.

With continued reference to FIGS. 2 and 3, in some examples, each medical device 12A-12N (or grouping of medical devices 12B-12N integrated with the patient table 12A) may include a medical device location identifier. In this manner, as an individual with a location identifier 18A-18N crosses the predetermined range D1-DN, the control system 100 (e.g., the processor 104) receives a detection from the monitoring system 14 and determines which classification C1-CN is associated with the individual. Based on the classifications C1-CN, various features and available settings become available on some or all of the medical devices 12A-12N. For example, an individual (e.g., caregiver) with the first caregiver classification C1 may have limited access to certain features. For example, the first caregiver classification C1 may be associated with a nursing assistant that has the primary responsibility of changing a position of the patient table 12A (e.g., via articulation of the linkages 30, movement of the central pedestal 36, and/or a locked or drivable condition of the caster wheels 34), checking a patient weight (e.g., with the weight sensor 12B), periodically checking vitals (e.g., via the vitals monitor 12C), checking a fluid exchange rate (e.g., via the fluid delivery system 12D). However, in many cases, the individual with the first caregiver classification C1 may not have certain responsibilities, such as the measurement and delivery of medication (e.g., via the drug delivery system 12E). In such cases, the control system 100 (e.g., the processor 104) may prevent use and/or monitoring of the drug delivery system 12E when there are no individuals with accepted second or third classifications C2, C3 within the predetermined range D1-DN. Within a predetermined range D1-DN may be defined as below the predetermined range D1-DN (e.g., a caregiver is in close proximity to the medical device 12A-12N and certain features are enabled), above the predetermined range D1-DN (e.g., a caregiver leaves proximity to the medical device 12A-12N and certain features are disabled), and/or combinations thereof.

With continued reference to FIGS. 2 and 3, an individual with the second caregiver classification C2 may be associated with a registered nurse that has primary responsibilities beyond and/or different than the first caregiver classification C1 classification. In some examples, the individual with the second caregiver classification C2 may have access to the same settings as the individual with the first caregiver classification C1. In addition, the individual with the second caregiver classification C2 may have access to additional settings, such as changing the fluid exchange rate (e.g., via the fluid delivery system 12D) and/or getting access to the measurement and delivery of medication (e.g., via the drug delivery system 12E).

With continued reference to FIGS. 2 and 3, an individual with the third caregiver classification C3 may be associated with a medical doctor (M.D.) or other specialty caregiver that has primary responsibilities beyond and/or different than both the first and second caregiver classifications C1 and C2. In some examples, the individual with the third caregiver classification C3 may have access to the same settings as the individuals with the first and second caregiver classifications C1 and C2. In addition, the individual with the third caregiver classification C3 may have access to additional settings, such as adding medication to the drug delivery system 12E and adjusting a rate of delivery of the medication.

With continued reference to FIGS. 2 and 3, an individual with the visitor classification C4 or the non-care providing staff classification C5 may not have or have limited access to any settings on the medical devices 12A-12N. For example, an individual with the visitor classification C4 may not have access to any of the settings of the medical devices 12A-12N while an individual with the non-care providing staff classification C5 may have access to certain features like moving the patient table 12A (e.g., for cleaning). In some examples, the individual with the non-care providing staff classification C5 may only have access to various features if no patient is present (e.g., as detected via the weight sensor 12B or other features of the monitoring system 14). In this manner, the patient is protected from unrestricted access to the medical devices 12A-12N from untrained and/or inappropriate users. An individual with a priority user classification C6 may have unique responsibilities that can be individualized for specific access and use of the medical devices 12A-12N. In some examples, a patient classification C7 may be assigned. In some examples, each individual with a classification C1-CN may have a unique personnel location identifier 18A-18N, such that the adaptive healthcare system 10 can monitor occupants on an individual basis. In some examples, access to various features and settings of the medical devices 12A-12N may be inaccessible by default unless an individual with an appropriate classification C1-CN is within the predetermined range D1-DN.

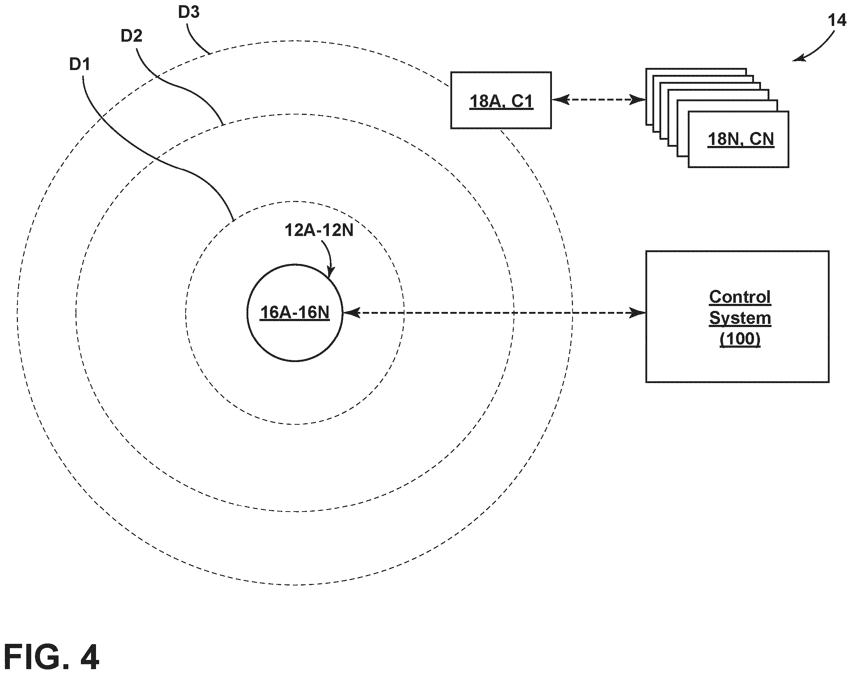

With reference now to FIGS. 3 and 4, the medical facility 46 may include a plurality of rooms 50A-50F. Each room 50A-50F may have different medical devices 12A-12N and/or patient needs. The rooms 50A-50F may be connected by a hallway 52 and accessible via doors 53. In some implementations, specific classifications C1-CN are needed to unlock one or more of the doors 53. The adaptive healthcare system 10 (e.g., the monitoring system 14) may be utilized across the medical facility 46 (e.g., multiple or all of the rooms 50A-50F and the hallway 52). Accordingly, it should be appreciated that the predetermined range D1-DN of proximity may be limited to individual rooms 50A-50F in which the associated medical device 12A-12N is located. In this manner, an individual in an adjacent room with a higher classification may not enable use or access to settings of a medical device 12A-12N in a room that they do not occupy. In this manner, a person with an unqualified classification C1-CN cannot access the settings of the medical devices 12A-12N if a person with a qualified classification C1-CN is not in the room.

With reference now to FIGS. 1-4, a visual indicator 54 may be associated with one or more of the medical devices 12A-12N. The visual indicator 54 may be a light located on a medical device 12A-12N or other locations within the rooms 50A-50F (FIG. 2), a message on the display 44 of the user interface 40. The visual indicator 54 may be illuminated to communicate certain conditions of a patient, such as vitals, a patient's need for a healthcare provider, and/or the like. In some examples, the display 44 may be configured to be one of off or dimmed in the power saving mode and illuminated in the power unrestricted mode. In this manner, additional power saving can be implemented.

With reference now specifically to FIG. 4, the predetermined range D1-DN may be varied and dependent on the medical devices 12A-12N and settings associated therewith. For example, FIG. 4 illustrates predetermined ranges D1-D3, however, any number of additional predetermined ranges DN may be implemented. Predetermined ranges D1-D3 may include a first predetermined range D1 located nearest one of the medical devices 12A-12N, a second predetermined range D2 located second nearest the medical device 12A-12N, and a third predetermined range D3 located the furthest from the medical device 12A-12N. Various methods may be implemented by the control system 100 (e.g., the processor 104) to permit the use, provide settings, and access to the medical devices 12A-12N based on certain conditions. For example, in some scenarios, such as the administration of medicine (e.g., anesthetics) via the drug delivery system 12E may only be allowed by an individual with the third caregiver classification C3. Because the improper administration of medicine can be particularly dangerous to a patient, the individual with the third caregiver classification C3 may have to be particularly close (e.g., the first predetermined range D1) to the drug delivery system 12E before administration settings are available. On the other hand, reviewing a delivery rate without having access to modifying the delivery rate, may become available at predetermined ranges D2 and D3 at further distances. Further, in some implementations, once the predetermined range D1-D3 is accessed by a qualified person, the available settings may be accessed for a predefined amount of time after the qualified caregiver leaves the predetermined range D1-D3 (e.g., for a minute, five minutes or less, etc.). In some implementations, once the predetermined range D1 is accessed by a qualified person, the available settings may be accessed until the qualified caregiver leaves the predetermined range D3. In this manner, the qualified caregiver can move around the room 50A-50F after the initial permissions are granted to, for example, train individuals on the operation of the medical device 12A-12N and/or otherwise move about the room 50A-50F for supplies, tending to the patient, or washing their hands.

With reference now specifically to FIG. 4, in some scenarios, it may be approved by medical governance rules and regulations to permit individuals with a restricted classification to have additional responsibilities when in the presence of an individual with a senior, less restricted or unrestricted classification (e.g., oversight classification). For example, an individual with the first caregiver classification C1 may typically restricted from changing the fluid exchange rate (e.g., via the fluid delivery system 12D) unless they are in the presence of an individual with the second caregiver classification C2. In this manner, the control system 100 (e.g., the processor 104) may detect the individual with the first caregiver classification C1 within a predetermined range D1-DN and, in response, detecting if the individual with the second caregiver classification C2 is also within a predetermined range D1-DN. In some examples, the predetermined range D1-DN required for the individual with the oversight classification (e.g., the second caregiver classification C2) may be greater in distance than that required when the individual with the oversight classification is performing the tasks associated with the medical device 12A-12N themselves. In this manner, the management of medical facility 46 can be precisely controlled based on medical governance rules and regulations.

In some examples, the control system 100 may require the individual with the oversight classification to initially enter the predetermined range D1 prior to allowing the individual with the first caregiver classification C1 to have more access to the previously restricted settings. After the initial confirmation that the individual with the oversight classification has entered the predetermined range D1, the control system 100 may permit the individual with the oversight classification to, for example, leave the predetermined range D1 into the predetermined range D2 while still permitting access to previously restricted settings the individual with the first caregiver classification C1. However, once the individual with the oversight classification leaves the predetermined range D3, the control system 100 may generate an alert for the individual with the oversight classification to move closer or restrict the settings of the medical device 12A-12N. The alert may be timed, where the individual with the oversight classification is given a time limited window (e.g., one minute or less, five minutes or less) to re-enter the predetermined range D2 before restricting the settings of the medical device 12A-12N. While changing the fluid exchange rate (e.g., via the fluid delivery system 12D) is provided as an example, it should be appreciated that the same principles may be applied to each of the medical device 12A-12N, for example, delivery of medication (e.g., via the drug delivery system 12E).

In some implementations, the adaptive healthcare system 10 includes one or more of the medical devices 12A-12N that have a plurality of settings. The monitoring system 14 includes a device location identifier 16A-16N and a pair of personnel location identifiers 18A-18N (e.g., caregiver location identifiers 18A-18N) that include a caregiver classification and an oversight classification. The processor 104 may be caused to detect that a proximity between the device location identifier 12A-12N and the personnel location identifier associated with the caregiver classification 18A-18N is within a predetermined range D1-D2. The processor 104 is further caused to enable a restricted plurality of settings of the medical device 12A-12N based on a combination of the proximity and the caregiver classification 18A-18N and detect if a proximity between the device location identifier 16A-16N and the personnel location identifier 18A-18N associated with the oversight classification is within a predetermined range D1-D2. If the personnel location identifier 18A-18N associated with the oversight classification is within a predetermined range, the processor 104 then enables additional plurality of settings of the medical device.

With reference now to FIGS. 1-4, the monitoring system 14 may utilize various technologies for the plurality of device location identifiers 16A-16N and the plurality of personnel location identifiers 18A-18N. In some examples, the device location identifiers 16A-16N and the personnel location identifiers 18A-18N may be device-based, such as transmitters, receivers, and/or transceivers. For example, the devices may be configured for short-range communications, such as Radio-Frequency Identification (RFID), Bluetooth, Wi-Fi, near-field communication (NFC), LPWAN, ultra-wideband (UWB), IEEE 802.15, a combination thereof, and/or the like. The devices may be RFID tags, personal mobile device (e.g., cell phones), or other types of technology. In some examples, the device location identifiers 16A-16N and the personnel location identifiers 18A-18N may be digitally-based, such as via image-based recognition tools. For example, image-based recognition tools may include cameras with deep neural network learning algorithms that may determine a classification C1-CN of an individual through physical appearance, clothing, markings on clothing or keycards, and/or the like. In some examples, both device-based and image-based technologies may be used concurrently. In some examples, the communication between the plurality of device location identifiers 16A-16N and the plurality of personnel location identifiers 18A-18N may be communicated to the control system 100 and saved as metadata for oversight and record keeping.

FIG. 5A illustrates the control system 100 associated, for example, with the one or more of the medical device 12A-12N, the monitoring system 14, the user interface 40, the visual indicator 54, and other components of the adaptive healthcare system 10. As will be appreciated with further reading, the control system 100 may also be associated with and/or receive instructions from a computer program product 150 that includes instructions to carry out the methods and functionalities described herein. The control system 100 may include an electronic control unit (ECU) 102. The ECU 102 may include the processor 104 and a memory 106. The processor 104 may include any suitable processor 104. Additionally, or alternatively, the ECU 102 may include any suitable number of processors, in addition to or other than the processor 104. The memory 106 may comprise a single disk or a plurality of disks (e.g., hard drives), and includes a storage management module that manages one or more partitions within the memory 106. In some examples, the memory 106 may include flash memory, semiconductor (solid state) memory, a database accessible via a cloud, a combination thereof, and/or the like. The memory 106 may include Random Access Memory (RAM), a Read-Only Memory (ROM), or a combination thereof. The memory 106 may include instructions that, when executed by the processor 104, cause the processor 104 to, at least, perform the functions and method steps as described herein. In some examples, the memory 106 may include at least some instructions received from the computer program product 150. In some examples, the control system 100 is a global control system in communication with a plurality of ECUs 102 associated with the various medical devices 12A-12N and/or other components of the adaptive healthcare system 10. In some examples, the control system 100 is a local control system associated with one of medical devices 12A-12N and/or other components of the adaptive healthcare system 10. In some examples, components of the control system 100 are both local and remote (e.g., global) and in communication with one another. In other words, the adaptive healthcare system 10 may be implemented locally for use with individual medical devices 12A-12N and/or globally for a plurality of the medical devices 12A-12N and/or other components of the adaptive healthcare system 10 located throughout the medical facility 46 or outside of the medical facility 46.

With continued reference to FIG. 5A, the ECU 102 may receive (e.g., from the intranet) and/or the memory 106 may contain software 108, a classification dictionary 110, a medical device setting dictionary 112, a protocol dictionary 114, a system log 116, and a power management protocol 118. More particularly, the software 108 may have updatable instructions that operate various features of the adaptive healthcare system 10 in a remote or locally based application. The software 108 may include instructions for generating global statistics about the adaptive healthcare system 10 or local statistics related to a single medical device 12A-12N or single user. In some examples, the software 108 may generate statistics related to a single classification of medical devices 12A-12N, personnel, or locality within the medical facility 46.

The classification dictionary 110 may include a list of personnel and their associated classification C1-CN, which may be updated via the user interface 40 or a remote computing device 56 (FIG. 3). The medical device setting dictionary 112 may include a list of allowed settings for medical devices 12A-12N based on classification C1-CN and may further include a location in the medical facility 46 where each of the medical devices 12A-12N are located. The protocol dictionary 114 may include a list of the predetermined ranges D1-DN and specific allowable use cases of an individual with a junior classification having additional access to settings when in the presence of an individual with a senior classification. The system log 116 may include a list of interactions between personnel and the predetermined ranges D1-DN as well as interactions between personnel and the medical devices 12A-12N. The system log 116 may further include timestamps of interactions and personal identifying information on the personnel involved in the interactions. The system log 116 may further include attempted access to the plurality of settings, the classification C1-CN of the detected caregiver, and if the classification C1-CN of the detected caregiver is authorized to the access the plurality of settings.

The power management protocol 118 may include procedures for power usage of the medical devices 12A-12N (e.g., power systems) and the visual indicators 54. For example, the user interface 40 (e.g., the display 44) and the visual indicators 54 may only be actuated when an individual with an appropriate classification C1-CN is within one of the predetermined ranges D1-DN associated having visibility of the display 44 or visual indicator 54. Likewise, certain settings of the various medical devices 12A-12N may not be needed unless the individual with an appropriate classification C1-CN is within one of the predetermined ranges D1-DN. For example, the adjustment of the patient bed 12A, active weight sensor 12B measurements, displaying vital measurements from the vitals monitor 12C, and/or displaying flow rate of the fluid delivery system 12D or drug delivery system 12E. Similarly, the power management protocol 118 may include instructions for continuing to provide power of certain features related to the medical devices 12A-12N, such as continued monitoring of vitals and flow rates of the fluid delivery system 12D or drug delivery system 12E independent of an individual's proximity or classification C1-CN. In some examples, measurements associated with the medical devices 12A-12N may still be displayed on the remote computing device 56 regardless of the status of the medical device 12A-12N and an individual's proximity or classification C1-CN. Generally speaking, the power management protocol 118 saves power usage by selectively reducing power to non-critical features of the medical devices 12A-12N and visual indicators 54 when not in use and continuing to power the critical features of the medical devices 12A-12N. The power saving both reduces the power consumption of the power systems (e.g., power system 41) and the prolonged usage of batteries 42.

It should be appreciated that the saved instructions 108-118 may be modified globally to the medical facility 46 based on changes to personnel classification C1-CN, updates to medical governance rules and regulations, the presence of additional personnel, and/or the like. The non-critical features may include features associated with the medical device 12A-12N rather than specifically the user interface 40. For example, the non-critical feature may include the generation and transmission of a visualization of a delivery rate of a medication on the user interface 40. The non-critical feature may further or alternatively include the weight sensor 12B in a patient table 12A, the adjustment mechanism (e.g., the central pedestal 36) on the patient table 12A, and other features which are not necessary for patient care while a caregiver is absent.

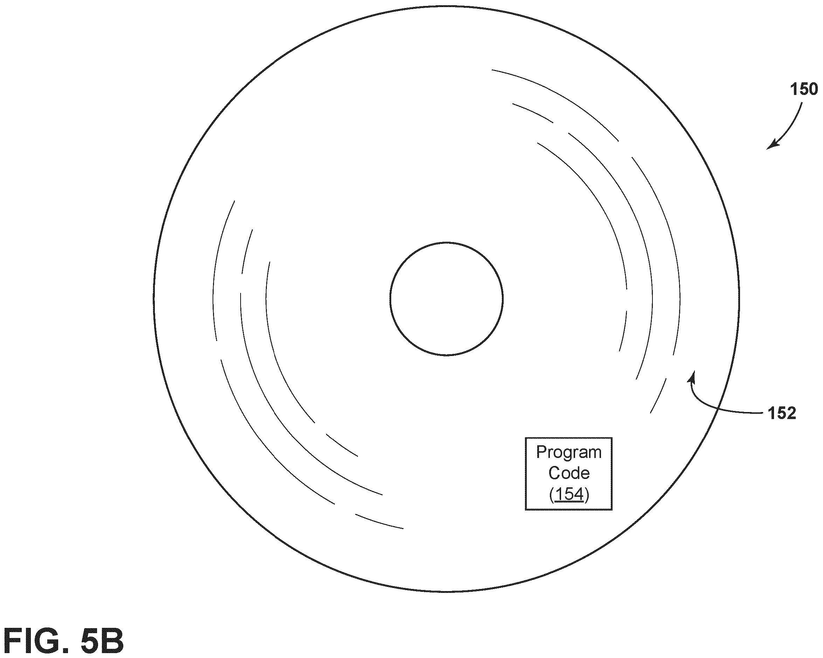

With reference now to FIG. 5B, in one example, the aforementioned computer program product 150 may include many of the instructions associated with the memory 106. More particularly, the instructions included in the computer program product 150 may include instructions (e.g., saved in non-transitory memory) related to the methods and functionalities described herein and carried out by the control system 100. For example, the computer program product 150 may include instructions to perform the method steps and functions described herein, but may rely on certain features/inputs such as from the medical devices 12A-12N, the device location identifiers 16A-16N, and the personnel location identifiers 18A-18N (e.g., caregiver location identifiers 18A-18B) that each include one of a first caregiver classification C1 and a second caregiver classification C2. The computer program product 150 may further include select or all features of the software 108, a classification dictionary 110, a medical device setting dictionary 112, a protocol dictionary 114, a system log 116, and a power management protocol 118. In some examples, the computer program product 150 may include instructions for the adaptive healthcare system 10 to collect and store certain features/inputs such as from the medical devices 12A-12N, the device location identifiers 16A-16N, and the personnel location identifiers 18A-18N.

The computer program product 150 may include, for instance, one or more computer readable medium 152 (e.g., non-transitory memory) to store computer readable program code means or logic 154 in order to provide and facilitate one or more functions and methods steps described in the present disclosure. The program code contained or stored in/on a computer readable medium 152 can be obtained and executed by a computer, such as the control system 100 to behave/function in a particular manner. The program code can be transmitted using any appropriate medium, including (but not limited to) wireless, wireline, optical fiber, and/or radio-frequency. The program code 154 includes instructions for carrying out operations to perform, achieve, or facilitate aspects of the disclosure may be written in one or more programming languages. In some examples, the programming language(s) include object-oriented and/or procedural programming languages such as C, C++, C#, Java, and/or the like. The program code 154 may execute entirely on the control system 100, locally and/or globally (e.g., remotely). In some examples, the local and global components of the control system 100 are in communication via a network such as a local area network (LAN) or a wide area network (WAN), and/or via an external computer (for example, through the Internet using an Internet Service Provider).

In one example, the program code 154 includes one or more program instructions obtained for execution by one or more processors (e.g., the processor 104). The instructions contained on the program code 154 may be provided to the one or more processors of, for example, one or more computer systems (e.g., e.g., the remote computing device 56), such that the program instructions, when executed by the one or more processors, perform, achieve, or facilitate aspects of the functionalities and methods described herein. The descriptions, FIGS. and, flowcharts depicted and described herein illustrate the architecture, functionality, and operation of possible examples of system 10, methods, and/or the computer program product 150 according to an aspect of the present disclosure.

In some examples, as noted above, descriptions, actions, functions, FIGS. and, flowcharts depicted and described herein may represent and/or be the result of a module, segment, or portion of code, which includes one or more executable instructions for implementing the specified behaviors and/or logical functions. It should be appreciated that actions, functions, blocks described herein may occur in a different order than depicted and/or described, or may occur simultaneous to, or partially/wholly concurrent with, one or more actions, functions, or blocks. For example, two actions or method steps (e.g., blocks) shown and/or described in succession may, in fact, be executed substantially concurrently or in the reverse order.

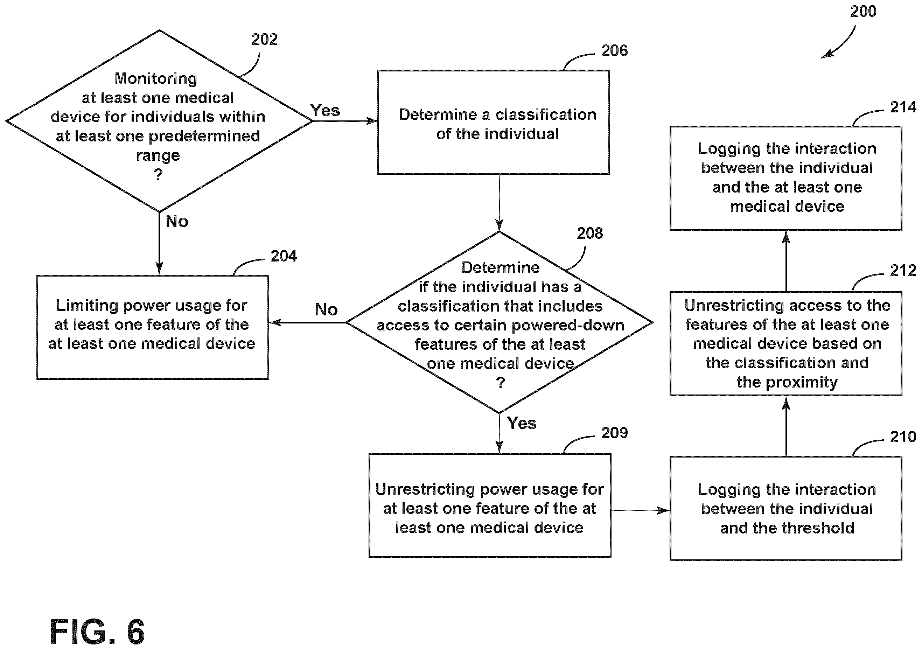

FIG. 6 illustrates a method 200 of utilizing an adaptive healthcare system 10 that may be, for example, carried out by the control system 100 or the control system 100 receiving instructions from the computer program product 150. At step 202, the method 200 includes monitoring at least one medical device 12A-12N for individuals within at least one distance predetermined range D1-DN (e.g., the furthest predetermined range DN). For example, a monitoring system 14 may be utilized to detect a device location identifier 16A-16N and a personnel location identifier 18A-18N. At step 204, if no individual is within the at least one distance predetermined range D1-DN, the method 200 includes limiting power usage for at least one feature of the at least one medical device 12A-12N. For example, the control system 100 (e.g., the processor 104) may be instructed by the power management protocol 118 to reduce power to non-critical features of the at least one medical device 12A-12N in addition to visual indicators 54. At step 206, if an individual is within the at least one distance predetermined range D1-DN, the method 200 includes determining a classification C1-CN of the individual. For example, individuals with different classifications C1-CN may have access to various features of the at least one medical device 12A-12N. At step 208, if the individual has a classification C1-CN that includes access to certain powered-down features of the at least one medical device 12A-12N, power usage becomes unrestricted at step 209 for those features of the at least one medical device 12A-12N. For example, the power management protocol 118 instructs the processor 104 to generate a signal to the at least one medical device 12A-12N. At step 210, the interaction between the individual and the predetermined range D1-DN is logged. For example, the interaction between the individual and the predetermined range D1-DN as well as the individual classification C1-CN may be saved in the system log 116. At step 212, the method 200 includes unrestricted access to the features of the at least one medical device 12A-12N based on the classification C1-CN and the proximity (e.g., predetermined range D1-DN). For example, the processor 104 may receive instructions from the classification dictionary 100 to determine the classification C1-CN, the medical setting dictionary 112 to determine which settings are available based on the assigned classification C1-CN, and the protocol dictionary 114 to determine if the individual is within an appropriate predetermined range D1-DN to use or modify the settings and features. It should be appreciated that if the individual has a low classification (e.g., a non-care provider), the settings and features may remain unrestricted and/or in the powered-down status. Step 212 may further include determining if a second individual with a permissible oversight classification is present. If the second individual is present, then the method 200 may include allowing additional access to features that are permissible for the first individual to utilize while in the presence of the second individual with the oversight classification. At step 214, the interaction between the individual and the at least one medical device 12A-12N is logged. For example, the interaction between the individual and the at least one medical device 12A-12N as well as the individual's classification C1-CN may be saved in the system log 116.

The disclosure herein may be further summarized in the following paragraphs and further characterized by combinations of any and all of the various aspects described therein.

According to one aspect of the present disclosure, an adaptive healthcare system includes at least one medical device having a user interface with a plurality of settings, the user interface includes a power saving mode and a power unrestricted mode. A monitoring system includes a device location identifier and a plurality of caregiver location identifiers that include at least one of a first caregiver classification and a second caregiver classification. The adaptive healthcare system further includes a processor and memory containing instructions that, when carried out by the processor, cause the processor to perform various functions. More particularly, the processor is caused to detect that a proximity between the device location identifier and one of the plurality of caregiver location identifiers is within a predetermined range and switches the user interface from the power saving mode to the power unrestricted mode. The processor is further caused to determine if the detected caregiver location identifier includes the first caregiver classification or the second caregiver classification. The processor is further caused to enable at least one of the plurality of settings of the medical device based on a combination of the proximity and the determined classification of the detected caregiver location identifier.

The processor may be further caused to enable a first plurality of settings for the first caregiver classification and a second plurality of settings that includes all the first plurality of settings and additional settings for the second caregiver classification.

At least one of a first and second caregiver classifications may include a plurality of additional caregiver classifications with access to different pluralities of settings than the first and second plurality of settings.

The second caregiver classification may be associated with an individual with greater responsibilities to a patient than the first caregiver classification, and the plurality of additional caregiver classifications includes a third caregiver classification that is associated with an individual with greater responsibilities to the patient than the second caregiver classification.

The at least one medical device may include a patient table.

The user interface may include a display that is one of off or dimmed in the power saving mode and illuminated in the power unrestricted mode.

The plurality of settings may be displayed on the user interface in the power unrestricted mode.

The at least one medical device may include a drug delivery system.

The plurality of settings may include monitoring a delivery rate of a medication and adjusting the delivery rate of the medication.

The first caregiver classification may enable the monitoring of the delivery rate of the medication but restricts adjusting the delivery rate of the medication and the second caregiver classification enables the monitoring of the delivery rate of the medication and adjustment of the delivery rate of the medication.

The memory may include a power management protocol that causes the processor to reduce power to a non-critical feature of the at least one medical device, and upon determining if the detected caregiver location identifier includes the first caregiver classification or the second caregiver classification, unrestricting the power to the non-critical feature associated with the at least one of the plurality of settings that are enabled. In this example, the at least one medical device may include a drug delivery system and the non-critical feature may include a visualization of a delivery rate of a medication on the user interface. The non-critical feature may include of further include an adjustment mechanism in a patient table. The non-critical feature may include or further include a weight sensor in a patient table.

The processor may be further caused to log any interactions between the detected caregiver location and the at least one medical device.

According to a further aspect of the present disclosure, an adaptive healthcare system for a medical facility having a room includes a visual indicator configured to be located in the room and to communicate a need of a patient. A medical device is configured to be located in the room and includes a plurality of settings. A monitoring system includes a device location identifier and a plurality of personnel location identifiers that each include one of a plurality of classifications. The adaptive healthcare system further includes a processor and memory, the memory containing instructions that when carried out by the processor, cause the processor to detect that a proximity between the device location identifier and one of the plurality of personnel location identifiers is within a predetermined range that is within view of the visual indicator. The processor is further caused to determine that the personnel location identifier includes a classification with a responsibility associated with the need of the patient and illuminate the visual indicator while the detected personnel location identifier is within the predetermined range.

The processor may be further caused to turn the visual indicator off once the detected personnel location identifier moves outside of the predetermined range.

The memory may include a power management protocol that causes the processor to reduce power to a non-critical feature of the medical device and, upon determining if the detected personnel location identifier includes one of the plurality of classifications with responsibilities associated with the non-critical feature, unrestricting the power to the non-critical feature. The non-critical feature may include a display of a user interface.

The processor may be further caused to log any interactions between the detected caregiver location and the non-critical feature of the medical device.

According to a yet further aspect of the present disclosure, an adaptive healthcare system includes a medical device including a plurality of settings. A monitoring system includes a device location identifier and a pair of personnel location identifiers that include a caregiver classification and an oversight classification. The adaptive healthcare system further includes a processor and memory, the memory containing instructions that when carried out by the processor, cause the processor to detect that a proximity between the device location identifier and the personnel location identifier associated with the caregiver classification is within a predetermined range. The processor is further caused to enable a restricted plurality of settings of the medical device based on a combination of the proximity and the caregiver classification and detect if a proximity between the device location identifier and the personnel location identifier associated with the oversight classification is within a predetermined range. If the personnel location identifier associated with the oversight classification is within a predetermined range, the processor enables additional plurality of settings of the medical device.

The processor may be further caused to log any interactions between the detected personal location and the medical device.

It will be understood by one having ordinary skill in the art that construction of the described disclosure and other components is not limited to any specific material. Other examples of the disclosure disclosed herein may be formed from a wide variety of materials, unless described otherwise herein.

For purposes of this disclosure, the term "coupled" (in all of its forms, couple, coupling, coupled, etc.) generally means the joining of two components (electrical or mechanical) directly or indirectly to one another. Such joining may be stationary in nature or movable in nature. Such joining may be achieved with the two components (electrical or mechanical) and any additional intermediate members being integrally formed as a single unitary body with one another or with the two components. Such joining may be permanent in nature or may be removable or releasable in nature unless otherwise stated.

It is also important to note that the construction and arrangement of the elements of the disclosure, as shown in the examples, is illustrative only. Although only a few examples of the present innovations have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter recited. For example, elements shown as integrally formed may be constructed of multiple parts, or elements shown as multiple parts may be integrally formed, the operation of the interfaces may be reversed or otherwise varied, the length or width of the structures and/or members or connector or other elements of the system may be varied, the nature or number of adjustment positions provided between the elements may be varied. It should be noted that the elements and/or assemblies of the system may be constructed from any of a wide variety of materials that provide sufficient strength or durability, in any of a wide variety of colors, textures, and combinations. Accordingly, all such modifications are intended to be included within the scope of the present innovations. Other substitutions, modifications, changes, and omissions may be made in the design, operating conditions, and arrangement of the desired and other exemplary embodiments without departing from the spirit of the present innovations.

It will be understood that any described processes or steps within described processes may be combined with other disclosed processes or steps to form structures within the scope of the present disclosure. The exemplary structures and processes disclosed herein are for illustrative purposes and are not to be construed as limiting.

## Claims

1. An adaptive healthcare system (10) comprising:
at least one medical device (12A-12N) including a user interface (40) with a plurality of settings, the user interface (40) having a power saving mode and a power unrestricted mode;
a monitoring system (14) including:
a device location identifier (16A-16N); and
a plurality of caregiver location identifiers (18A-18N) that include at least one of a first caregiver classification (C1) and a second caregiver classification (C2);
a processor (104) and memory (106), the memory (106) containing instructions that when carried out by the processor (104), cause the processor (104) to:
detect that a proximity between the device location identifier (16A-16N) and one of the plurality of caregiver location identifiers (18A-18N) is within a predetermined range (D1-DN);
switch the user interface (40) from the power saving mode to the power unrestricted mode;
determine if the detected caregiver location identifier (18A-18N) includes the first caregiver classification (C1) or the second caregiver classification (C2); and
enable at least one of the plurality of settings of the medical device (12A-12N) based on a combination of the proximity and the determined caregiver classification (C1, C2) of the detected caregiver location identifier.

2. The adaptive healthcare system (10) of claim 1, wherein the processor (104) is further caused to enable a first plurality of settings for the first caregiver classification (C1) and a second plurality of settings that includes all the first plurality of settings and additional settings for the second caregiver classification (C2).

3. The adaptive healthcare system (10) of claim 2, wherein the at least one of the first and second caregiver classifications (C1, C2) includes a plurality of additional caregiver classifications (C3-CN) with access to different pluralities of settings than the first and second plurality of settings.

4. The adaptive healthcare system (10) of claim 3, wherein the second caregiver classification (C2) is associated with an individual with greater responsibilities to a patient than the first caregiver classification (C1), and the plurality of additional caregiver classifications includes a third caregiver classification (C3) that is associated with an individual with greater responsibilities to the patient than the second caregiver classification (C2).

5. The adaptive healthcare system (10) according to any of the preceding claims, wherein the at least one medical device (12A-12N) includes a patient table (12A).

6. The adaptive healthcare system (10) according to any of the preceding claims, wherein the user interface (40) includes a display (44) that is one of off or dimmed in the power saving mode and illuminated in the power unrestricted mode.

7. The adaptive healthcare system (10) according to any of the preceding claims, wherein the plurality of settings are displayed on the user interface (40) in the power unrestricted mode.

8. The adaptive healthcare system (10) according to any of the preceding claims, wherein the at least one medical device (12A-12N) includes a medical device with a delivery rate critical to the support of the patient.

9. The adaptive healthcare system (10) of claim 8, wherein the plurality of settings includes monitoring the delivery rate and adjusting the delivery rate.

10. The adaptive healthcare system (10) as in claim 8 or claim 9, wherein the first caregiver classification (C1) enables the monitoring of the delivery rate but restricts adjusting the delivery rate and the second caregiver classification (C2) enables the monitoring of the delivery rate and adjustment of the delivery rate.

11. The adaptive healthcare system (10) according to any of the preceding claims, wherein the memory (106) includes a power management protocol that causes the processor (104) to:
reduce power to a non-critical feature of the at least one medical devices (12A-12N); and
upon determining if the detected caregiver location identifier (18A-18N) includes the first caregiver classification (C1) or the second caregiver classification (C2), unrestricting the power to the non-critical feature associated with the at least one of the plurality of settings that are enabled.

12. The adaptive healthcare system (10) of claim 11, wherein the at least one medical device (12A-12N) includes a drug delivery system (12E) and the non-critical feature includes a visualization of a delivery rate of a medication on the user interface (40).

13. The adaptive healthcare system (10) according to any of the preceding claims, wherein the processor (104) is further caused to log any interactions between the detected caregiver location and the at least one medical device (12A-12N) that includes attempted access to the plurality of settings, the caregiver classification (C1-CN) of the detected caregiver, and if the caregiver classification (C1-CN) of the detected caregiver is authorized to the access the plurality of settings.

14. An adaptive healthcare system (10) for a medical facility comprising:
a visual indicator configured be located in a room of the medical facility and to communicate a need of a patient;
a medical device configured to be located in the room and including a plurality of settings;
a monitoring system (14) including:
a device location identifier (16A-16N); and
a plurality of personnel location identifiers that each include one of a plurality of classifications;
a processor (104) and memory (106), the memory (106) containing instructions that when carried out by the processor (104), cause the processor (104) to:
detect that a proximity between the device location identifier (16A-16N) and one of the plurality of personnel location identifiers is within a predetermined range (D1-DN) that is within view of the visual indicator;
determine that the personnel location identifier includes a classification with a responsibility associated with the need of the patient; and
illuminate the visual indicator while the detected personnel location identifier is within the predetermined range (D1-DN).

15. The adaptive healthcare system (10) of claim 14, wherein the processor (104) is further caused to turn the visual indicator off once the detected personnel location identifier moves outside of the predetermined range (D1-DN).
